# EUROPEAN PATENT APPLICATION

(11) **EP 4 483 737 A1**
(43) Date of publication of application: **01.01.2025**
(21) Application number: 23182431.9
(22) Date of filing: 29.06.2023
(51) Int. Cl.: A41D 19/00, A61B 5/00, G01G 19/52, G08B 21/02

(54) **GLOVES FOR MONITORING THE MANUAL MOVEMENT OF LOADS BY A WORKER**

(71) Applicant: Cooperativa Edile Appennino Societa' Cooperativa a Responsabilita' Limitata, 33037 Pasian di Prato (UD) (IT)
(72) Inventor: MASCARO, Simone, 42025 CAVRIAGO RE (IT); GUZZO, Ciro, 42124 REGGIO EMILIA (IT)
(74) Representative: Modiano, Micaela Nadia

(57) **Abstract**

Gloves (1) for monitoring the manual movement of loads by a worker, which comprise means (2) configured to detect pressure which are controlled by a control and management assembly (3).

The control and management assembly (3) is configured to measure the weight of the load moved by the worker.

The weight is calculated on the basis of the variation in pressure detected by the means (2), with consequent emission of an alarm signal upon a detection of weight of the load moved by the worker greater than a predefined threshold level.

## Description

The present invention relates to gloves for monitoring the manual movement of loads by a worker.

It is known that labor activities to manually move loads place workers at risk of biomechanical overload injury, in particular back injury.

What is meant by manual movement of loads is operations to transport or support a load by one or more workers, including the actions of picking up, setting down, pushing, pulling, carrying or shifting a load, which, owing to their characteristics or as a consequence of unfavorable ergonomic conditions, entail risks of biomechanical overload injury, in particular back injury.

As clearly highlighted for example in Title VI of Legislative Decree no. 81/2008, an employer is required to adopt the necessary organizational measures and make use of appropriate means, in particular mechanical equipment, in order to avoid the need for manual movement of loads by workers.

If it is not possible to avoid manual movements, the employer must adopt the necessary organizational measures and provide the workers with adequate means, for the purpose of reducing the risk that the manual movement of these loads entails.

Examples of the above organizational measures may be, for example, subdividing the loads moved, raising/lowering the levels of gripping and setting down with the use of simple platforms, eliminating encumbrances in the work station, correctly informing and training workers on the lifting methods to be used, the use of suitable PPE such as gloves.

In some cases, unfortunately, these measures are not always correctly adopted by workers who, despite the training received, are either not capable of immediately understanding whether the load is heavier or lighter than expected, or they deliberately decide to ignore these measures, deeming them unnecessary, for example by not wearing gloves, which enable a secure and stable grip of the load to be moved, or by not separating the loads, as a consequence lifting a heavier load than that permitted.

Such actions, if repeated over time, can therefore entail biomechanical overload injuries for the workers.

Verification of the correct execution of the manual movement of loads, and verification of the use of gloves to do it, are currently the responsibility of the designated staff, in particular the manager of the risk prevention and protection service.

However, this verification method does not make it possible to have a complete and continuous monitoring of the workplace, such as for example of an industrial factory shed or of a construction site. It is in fact not always possible to have the constant presence of staff assigned to this role on-site, or in the factory shed, and in particular in every area thereof, at all times of the working day.

The aim of the present invention is to solve the above-mentioned drawbacks, by providing gloves for monitoring the manual movement of loads by a worker that enable the worker to verify whether he or she is moving a load of a weight that exceeds the permitted weight.

Within this aim, an object of the invention is to provide gloves for monitoring the manual movement of loads by a worker that enable the employer and the manager of the risk prevention and protection service to verify that the operations of manual movement of the loads are performed correctly by the individual workers.

Another object of the present invention is to provide gloves and a kit for monitoring the manual movement of loads by a worker, which are low-cost, easily and practically implemented, and safe in use.

This aim and these and other objects which will become more apparent hereinafter, are achieved by gloves for monitoring the manual movement of loads by a worker, which are characterized in that they comprise means configured to detect pressure which are controlled by a control and management assembly, said control and management assembly being configured to measure the weight of the load moved by the worker, calculated on the basis of the variation in pressure detected by said means, with consequent emission of an alarm signal upon a detection of weight of the load moved by the worker greater than a predefined threshold level.

Such aim and such objects are also achieved by a kit for monitoring the manual movement of loads, characterized in that it comprises:
- at least one pair of gloves provided with means configured to detect pressure and a transceiver block for sending a data string to a user interface module, said means and said block being controlled by a control and management assembly, said assembly being configured to measure the weight of the load moved by a worker calculated on the basis of the variation in pressure detected by said means;
- at least one user interface module such as a computer, a portable computer, a smartphone, a tablet computer and the like, provided with an apparatus for receiving signals;
- a software application configured to execute instructions for displaying and receiving text messages from said transceiver block;
said transceiver block of said gloves being adapted to send said data strings to said apparatus for receiving signals of said interface module, upon a detection of weight of the load moved by the worker greater than a predefined threshold level.

Further characteristics and advantages of the invention will become more apparent from the detailed description that follows of a preferred, but not exclusive, embodiment of the gloves for monitoring the manual movement of loads by a worker, according to the invention, which is illustrated by way of non-limiting example in the accompanying drawings wherein:

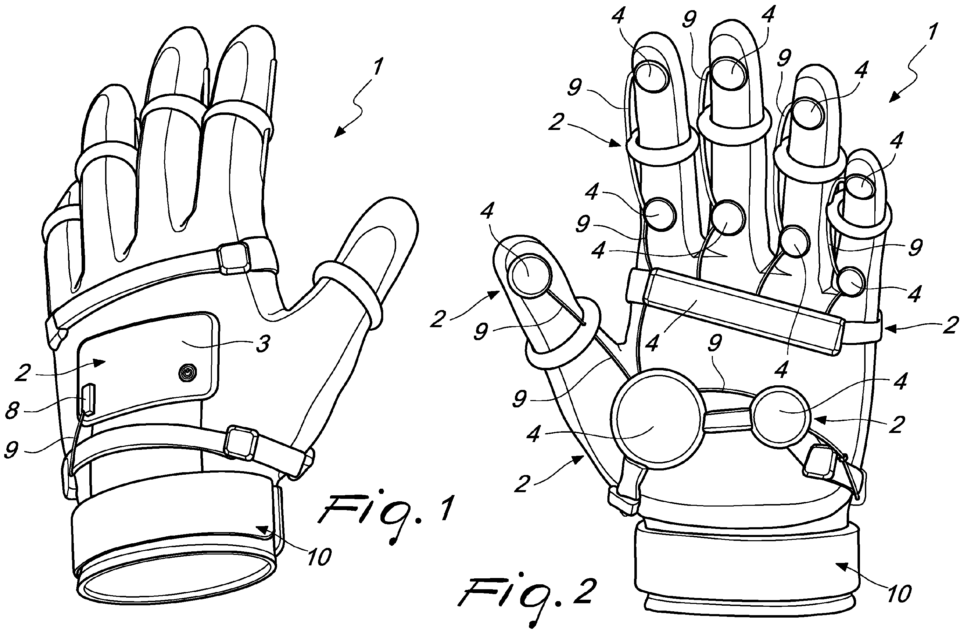

Figure 1 is a perspective view from above of the glove according to the invention;
Figure 2 is a perspective view from below of the glove according to the invention;
Figure 3 is a cross-sectional schematic view of a component of the gloves according to the invention;
Figure 4 is a cross-sectional schematic view of a component of the gloves according to the invention, with a load applied on the glove.

With reference to the figures, the reference numeral 1 generally designates gloves for monitoring the manual movement of loads by a worker, according to the present invention.

What is meant by manual movement of loads is operations to transport or support a load by one or more workers, including the actions of picking up, setting down, pushing, pulling, carrying or shifting a load, which, owing to their characteristics or as a consequence of unfavorable ergonomic conditions, entail risks of biomechanical overload injury, in particular back injury.

According to the invention, the gloves 1 comprise means 2 configured to detect pressure, which are controlled by a control and management assembly 3.

The control and management assembly 3 is configured to measure the weight of the load moved by the worker.

The weight is calculated on the basis of the variation in pressure detected by the means 2, with consequent emission of an alarm signal upon a detection of weight of the load moved by the worker greater than a predefined threshold level.

It should be noted that this threshold level can take account of different factors, i.e., the characteristics of the worker: sex, age, physical constitution, stature and the like; the characteristics of the load: shape, dimensions, center of gravity, graspability, stability and the like; the situation: distance and type of route to be taken, height and frequency of lifting, ambient temperature and the like.

It should be noted that generally, the maximum permitted weight of the load is comprised between 10 kg and 25 kg.

The means 2 can comprise at least one sealed pocket 4 containing a predefined amount of fluid. This fluid can preferably be air.

The pocket 4 can comprise a membrane 5 covering a modular supporting structure 6.

The membrane 5 can be made of a material preferably chosen from silicone, polymeric material, rubber and the like.

More specifically, the modular supporting structure 6 can be constituted by elements 7 of a shape preferably chosen from reticular, conical, cylindrical and the like.

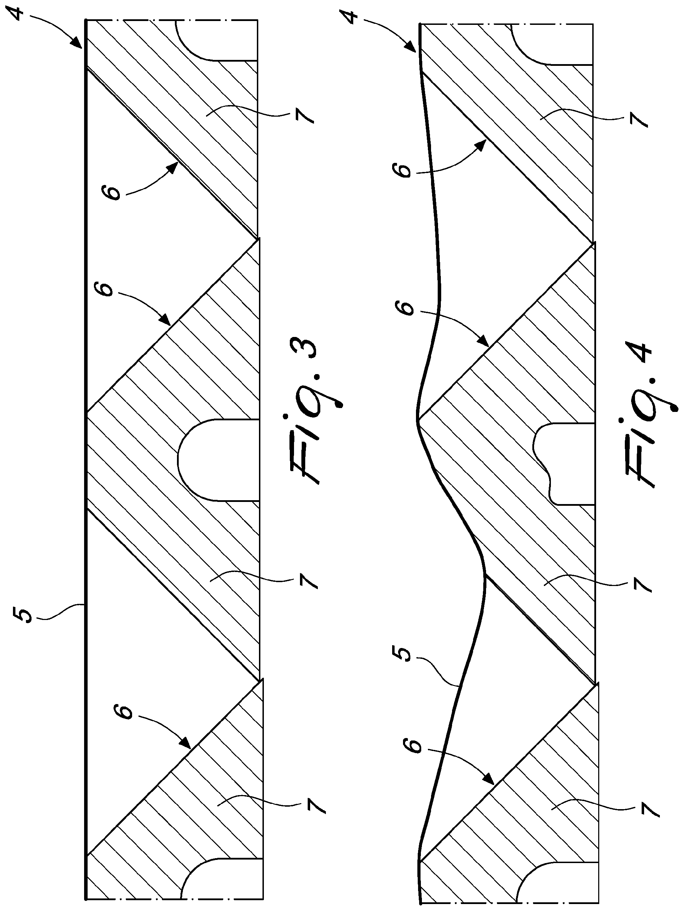

According to a solution of particular utility and practicality, illustrated in the accompanying Figure 3, the elements 7 can be conical, in order to recreate the elastic behavior of a conical spring.

It should be noted that the conical elements 7 can also have a recess 7a at the base in order to define a direction of deformation of the element and a predefined degree of deformability.

Furthermore, the conical elements 7 can comprise at least one sensor of electronic type in order to obtain further data so as to determine, with greater precision, the weight of the load lifted by the worker.

Furthermore, the means 2 can comprise at least one pocket 4 arranged respectively on each finger and on the palm of the gloves 1.

The possibility is not ruled out that the pocket 4 arranged on the palm can be larger in size than the pockets arranged on each finger.

Furthermore, on each finger there can be three pockets 4 which correspond respectively to the phalanx, middle phalanx and terminal phalanx of the gloves 1.

It should be noted that the thickness and the size of the single pockets 4 will be such as not to create encumbrance or annoyance for the worker.

The number, shape and dimensions of the pockets 4 may vary so that the gloves 1 can be adapted to any shape and size of the worker's hand.

The possibility is not ruled out that the means 2 can comprise a component 8 chosen from among a pressure sensor, a temperature sensor, an accelerometer, a gyroscope, a load cell and the like.

The component 8 can be controlled by the control and management assembly 3.

It should be noted that the presence of at least one component 8 chosen from among an accelerometer, a load cell and a gyroscope will make it possible to establish the position of the gloves 1 in space, in order to determine the direction in which the weight force is applied on the glove, and so determine with greater precision the weight of the load lifted by the worker.

Furthermore, the presence of the temperature sensor will prevent the measurement of the weight of the load being moved from being altered by any changes in the temperature of the surrounding environment.

For example, the worker might move a load between two places that are at appreciably different temperatures.

It should furthermore be noted that the means 2 can comprise a pneumatic circuit 9, made of non-deformable material, for connection between the pockets 4, the at least one component 8 and the control and management assembly 3.

This material can be polytetrafluoroethylene (PTFE).

The pressure exerted on the glove 1 by the load being moved, or the flexing of the hand, therefore will not affect the compression/elastic deformation of the pneumatic circuit 9, but only of the pockets 4. In fact, any compression of the circuit 9 could alter the detection of the pressure, and as a consequence the determination of the weight of the load being moved.

It should be noted that the tubes that constitute the pneumatic circuit 9 can have a diameter comprised between 0.5 mm and 1.5 mm.

According to a solution of particular effectiveness and efficiency, the gloves 1 can comprise an auxiliary power source 10.

The source 10 can be arranged on the back of the gloves 1, in order not to hamper the movement of the worker's hand and allow him or her to freely perform any activity.

Furthermore, the power source 10 can be of the type of a thin film battery, in order to reduce as far as possible both its weight and its bulk, while maintaining an operating time comprised between 8 and 10 hours.

According to a preferred solution, the gloves 1 can comprise at least one alert element preferably chosen from among an audio alarm, a vibrating apparatus, a transceiver block for sending a data string to a user interface module, a visual alarm and the like.

The alert element can be functionally associated with the control and management assembly 3.

More precisely, the user interface module can be of the type preferably chosen from a portable computer, a cellular telephone, a smartphone, a tablet computer, provided with: a software application configured to execute instructions for displaying and receiving text messages from the control and management assembly 3.

It should furthermore be noted that the control and management assembly 3 can be associated with at least one memory unit.

A plurality of data strings corresponding to individual worker profiles can be stored in this unit.

The possibility is not ruled out that each worker profile can then be associated with data of the type preferably chosen from an identification code, the task, the detections performed and the like.

From the point of view of construction, the gloves 1 can be made with electrically conductive fabrics, i.e. materials constituted by textile fibers that possess electrical conductivity, and in this manner it will be possible to stably position the means 2 configured to detect pressure, the control and management assembly 3, the auxiliary power source 10 and the alert element, all on the surface of the gloves 1, without it being necessary to have restraints that could obstruct the movement of the worker's hand and not allow him or her to freely perform any activity.

In addition to the gloves 1, the specific subject matter of the present discussion and of the protection claimed herein also relates to a kit for monitoring the manual movement of loads.

According to the invention, the kit for monitoring the manual movement of loads comprises at least one pair of gloves 1 provided with means 2 configured to detect pressure and a transceiver block for sending a data string to a user interface module.

The means 2 and the block can be controlled by a control and management assembly 3 which is configured to measure the weight of the load moved by a worker, which is calculated on the basis of the variation in pressure detected by the means 2.

The kit according to the invention also comprises at least one user interface module such as a computer, a portable computer, a smartphone, a tablet computer and the like, provided with an apparatus for receiving signals.

Furthermore, the kit comprises a software application configured to execute instructions for displaying and receiving text messages from the transceiver block.

The transceiver block of the gloves 1 is adapted to send data strings to the apparatus for receiving signals of the interface module, upon a detection of weight of the load moved by the worker greater than a predefined threshold level.

If in fact, the weight of the load lifted by the worker exceeds the permitted weight, the control and management assembly 3 sends an alarm message through the transceiver block to the user interface module, containing the alert that the load is not being correctly moved by the worker.

If this happens, the control and management assembly 3 will also automatically, and in real time, record the detected non-compliance in the memory unit.

So through the use of the gloves 1 according to the invention, the worker can have an immediate feedback, i.e., whether or not the weight of the load that he or she is moving is greater than a predefined threshold level.

Furthermore, the employer and/or the manager of the risk prevention and protection service will easily be able to monitor whether each worker is moving loads in the correct way.

Below is a possible method for using the kit 1, according to the invention.

Before proceeding to perform his or her specific work task, the worker puts on the gloves 1.

When the worker moves a load, as illustrated in Figure 3, a compression occurs of the pockets 4 on the gloves 1 that are in contact with the load.

This compression then generates an elastic deformation of the modular supporting structure 6 and a squashing of the membrane 5, at the gaps present in the structure 6, with consequent increase in the atmospheric pressure inside each single pocket 4.

The control and management assembly 3 will then measure the pressure detected by the pressure sensor in real time, in order to verify whether the weight of the load being moved complies with the current weight regulations.

If the weight is higher than the predefined threshold value, the control and management assembly 3 can, by way of the alert element, emit an audible and/or visual alarm and/or a vibration.

The worker, having received the alarm, can then either divide the load, thus avoiding the movement of an excessive weight, or use a lifting machine.

Furthermore, the control and management assembly 3 can send, through the transceiver block, an alarm message to the user interface module of the worker and/or of the safety manager and/or of the employer, containing an alert that the load being moved by the worker is heavier than the predefined threshold value.

According to a possible solution of particular utility and practicality, over the course of the working day the control and management assembly 3 and the transceiver block can be constantly active.

When the worker moves a load in an anomalous manner, the control and management assembly 3 can, by way of the alert element, emit an audible and/or visual alarm and/or a vibration and send one or more alarm messages to the user interface module of the worker who is committing the violation and/or of the safety manager and/or of the employer.

The control and management assembly 3 can record all the scans detected and store them, digitally signing them and applying a time stamp to them.

This makes it possible to produce documentation with a certain date, thus conferring legal value to it.

In fact, both the time stamp and the digital signature provide a guarantee over time that the archived files cannot be modified.

This aspect is particularly advantageous for the future.

In fact, if, after some years, a dispute should arise relating to a permanent disability caused by work, then by analyzing the archive of the scans it will be possible to determine what really happened.

In particular, it can be verified whether the worker always made correct use of the gloves 1, which are mandatory and made available by the employer.

For example, if a worker who for years performed a task that required the repeated and continual manual movement of loads, filed a claim for permanent invalidity owing to the type of work carried out, then it would be possible to verify, via an analysis of the archive of the scans, whether the employer actually fulfilled his or her responsibilities, by supplying adequate equipment and devices, and therefore whether he or she is responsible for what happened, or whether the worker instead did not fulfill his or her obligations, by not adopting the required precautions that would have ensured his or her safety.

The adoption of the kit 1 would therefore bring considerable tax advantages for the employer and a reduction in the costs of managing safety in the workplace.

Furthermore, the employer, alerted to the excessive weight being moved, can avoid a situation of excessive effort and of risk to his or her health that could, if repeated over time, result in the onset of biomechanical overload injury, in particular back injury.

Preventing this condition can also bring benefits from the psycho-/physical point of view, and a reduction in health costs to be sustained.

Advantageously, the gloves 1 for monitoring the manual movement of loads by a worker enable the worker to verify whether he or she is moving a load of weight that exceeds the permitted weight.

Usefully, the gloves 1 according to the invention make it possible for the employer and the manager of the risk prevention and protection service to verify that the operations of manual movement of the loads are performed correctly by the individual workers.

Profitably, the gloves 1 and the kit according to the invention are low-cost, easily and practically implemented and safe in use.

The invention thus conceived is susceptible of numerous modifications and variations, all of which are within the scope of the appended claims. Moreover, all the details may be substituted by other, technically equivalent elements.

In the embodiments illustrated, individual characteristics shown in relation to specific examples may in reality be interchanged with other, different characteristics, existing in other embodiments.

In practice, the materials employed, as well as the dimensions, may be any according to requirements and to the state of the art.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. Gloves for monitoring the manual movement of loads by a worker, which are **characterized in that** they comprise at least one position sensor and means (2) configured to detect pressure which are controlled by a control and management assembly (3), said control and management assembly (3) being configured to measure the weight of the load moved by the worker, calculated on the basis of the variation in pressure detected by said means (2), with consequent emission of an alarm signal upon a detection of weight of the load moved by the worker greater than a predefined threshold level.

2. The gloves according to claim 1, **characterized in that** said means (2) comprise at least one sealed pocket (4) containing a predefined amount of fluid, said pocket (4) comprising a membrane (5) covering a modular supporting structure (6).

3. The gloves according to claim 2, **characterized in that** said modular supporting structure (6) is constituted by elements (7) of a shape preferably chosen from reticular, conical, cylindrical and the like.

4. The gloves according to one or more of the preceding claims, **characterized in that** said means (2) comprise at least one pocket (4) arranged respectively on each finger and on the palm of said gloves (1).

5. The gloves according to one or more of the preceding claims, **characterized in that** said means (2) comprise at least one component (8) chosen from among a pressure sensor, a temperature sensor, an accelerometer, a gyroscope, a load cell and the like, said component (8) being controlled by said control and management assembly (3).

6. The gloves according to one or more of the preceding claims, **characterized in that** said means (2) comprise a pneumatic circuit (9) made of non-deformable material for connection between said pockets, said at least one component (8) and said management and control assembly (3).

7. The gloves according to one or more of the preceding claims, **characterized in that** they comprise an auxiliary power source (10), said source (10) being arranged on the back of said gloves (1).

8. The gloves according to one or more of the preceding claims, **characterized in that** comprise at least one alert element preferably chosen from among an audio alarm, a vibrating apparatus, a transceiver block for sending a data string to a user interface module, a visual alarm and the like, said alert element being functionally associated with said control and management assembly (3).

9. The gloves according to one or more of the preceding claims, **characterized in that** said user interface is of the type preferably chosen from a portable computer, a cellular telephone, a smartphone, a tablet computer, provided with a software application configured to execute instructions for displaying and receiving text messages from said transceiver block.

10. The gloves according to one or more of the preceding claims, **characterized in that** said control and management assembly (3) is associated with at least one memory unit, in said memory unit a plurality of data strings being stored which correspond to worker profiles, each profile being associated with data of the type preferably chosen from an identification code, a task, detections performed and the like.

11. A kit for monitoring the manual movement of loads, **characterized in that** it comprises:
- at least one pair of gloves (1) provided with means (2) configured to detect pressure and a transceiver block for sending a data string to a user interface module, said means (2) and said block being controlled by a control and management assembly (3), said assembly (3) being configured to measure the weight of the load moved by a worker calculated on the basis of the variation in pressure detected by said means (2);
- at least one user interface module such as a computer, a portable computer, a smartphone, a tablet computer and the like, provided with an apparatus for receiving signals;
- a software application configured to execute instructions for displaying and receiving text messages from said transceiver block;
said transceiver block of said gloves (1) being adapted to send said data strings to said apparatus for receiving signals of said interface module, upon a detection of weight of the load moved by the worker greater than a predefined threshold level.
